(19) Europäisches Patentamt
European Patent Office
Office européen des brevets

(11) Veröffentlichungsnummer : **0 038 438**
**B1**

(12) **EUROPÄISCHE PATENTSCHRIFT**

(45) Veröffentlichungstag der Patentschrift : **01.06.83**

(51) Int. Cl.³ : **A 61 K 31/41**// C07D271/08

(21) Anmeldenummer : **81102388.6**

(22) Anmeldetag : **30.03.81**

# (54) Substituierte 1,2,5-Oxadiazol-2-oxide zur Anwendung als Arzneimittel und sie enthaltende Arzneimittel.

(30) Priorität : **02.04.80 DE 3012862**

(43) Veröffentlichungstag der Anmeldung : **28.10.81 Patentblatt 81/43**

(45) Bekanntmachung des Hinweises auf die Patenterteilung : **01.06.83 Patentblatt 83/22**

(84) Benannte Vertragsstaaten : **AT BE CH DE FR GB IT LI NL SE**

(56) Entgegenhaltungen :
**IL FARMACO EDIZIONE SCIENTIFICA, Band XXVI, Nr. 3, März 1971, Seiten 241-246 Pavia, IT. A GASCO et al. : « Studi sulla chimica dell' 1,2,5-oxadiazolo (IV) (1) » Seiten 241-246**
**JOURNAL OF HETEROCYCLIC CHEMISTRY, 9, 1972, Seiten 577-580 U.S.A. A. GASCO et al. : « Synthesis and structure of some asymmetrically substituted furoxans. I. » Seiten 577-580**

(73) Patentinhaber : **CASSELLA Aktiengesellschaft**
**Hanauer Landstrasse 526**
**D-6000 Frankfurt am Main 61 (DE)**

(72) Erfinder : **Schönafinger, Karl, Dr.**
**Hünfelder Strasse 16**
**D-6000 Frankfurt 61 (DE)**
Erfinder : **Beyerle, Rudi, Dr.**
**An der Pfaffenmauer 44**
**D-6000 Frankfurt 60 (DE)**
Erfinder : **Mogilev, Anton, Dr.**
**Diaminstrasse 6**
**D-6000 Frankfurt 61 (DE)**
Erfinder : **Bohn, Helmut, Dr.**
**Kranzbergring 11**
**D-6369 Schöneck (DE)**
Erfinder : **Martorana, Piero, Dr.**
**Kaiser-Friedrich-Promenade 108**
**D-6380 Bad Homburg (DE)**
Erfinder : **Nitz, Rolf-Eberhard, Dr.**
**Heinrich-Bingemer-Weg 64**
**D-6000 Franfurt 60 (DE)**

(74) Vertreter : **Urbach, Hans-Georg, Dr. et al**
**Hanauer Landstrasse 526**
**D-6000 Frankfurt am Main 61 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Substituierte 1,2,5-Oxadiazol-2-oxide zur Anwendung als Arzneimittel und sie enthaltende Arzneimittel

Die vorliegende Erfindung betrifft die Verwendung von 1,2,5-Oxdiazol-2-oxiden der allgemeinen Formel I

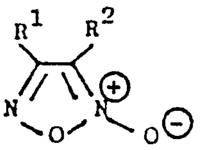

(I)

worin $R^1$ und $R^2$ die folgende Bedeutung besitzen :

| | $R^1$ | $R^2$ |
|---|---|---|
| 1. | CO—$NH_2$ | $CH_3$ |
| 2. | $COOC_2H_5$ | $CH_3$ |
| 3. | CO—$NHNH_2$ | $CH_3$ |
| 4. | COOH | $CH_3$ |
| 5. | —NH—$COOC_2H_5$ | $CH_3$ |
| 6. | —NH—$COOC_3H_7$(n) | $CH_3$ |
| 7. | —NH—$COOC_3H_7$(i) | $CH_3$ |
| 8. | —NH—$COOC_4H_9$(n) | $CH_3$ |
| 9. | —NH—$COOCH_2$-phenyl | $CH_3$ |
| 10. | CO—$NH_2$ | CO—$NH_2$ |
| 11. | CN | CN |
| 12. | COOH | $COOCH_3$ |
| 13. | $COOCH_3$ | $COOCH_3$ |
| 14. | $COOC_2H_5$ | $COOC_2H_5$ |
| 15. | $CH_3$ | CO—$NH_2$ |
| 16. | $CH_3$ | $COOC_2H_5$ |
| 17. | $CH_3$ | CO—$NHNH_2$ |
| 18. | $CH_3$ | COOH |
| 19. | CN | CO—$NH_2$ |
| 20. | CO—NH-phenyl | CO—NH-phenyl |
| 21. | $CH_3$ | NH—$COOC_2H_5$ |
| 22. | $CH_3$ | NH—$COOC_3H_7$(n) |
| 23. | $CH_3$ | NH—$COOC_3H_7$(i) |
| 24. | $CH_3$ | NH—$COOC_4H_9$(n) |
| 25. | $CH_3$ | NH—$COOCH_2$-phenyl |
| 26. | CO—NH-phenyl | $CH_3$ oder |
| 27. | $CH_3$ | CO—NH-phenyl |

bei der Bekämpfung oder der Vorbeugung von kardiovaskulären Erkrankungen.

Die vorstehenden Verbindungen der Formel I sind bekannt. Sie sind beispielsweise beschrieben in den folgenden Publikationen, in denen auch Verfahren zu ihrer Herstellung aufgeführt sind :

a) Ch. Grundmann, G. Nickel, R. K. Bansal, Liebigs Ann. Chem. *1975,* 1029 ;
b) A. Gasco, V. Mortarini, G. Ruá, E. Menziani, J. Het. Chem. *9,* 837, (1972) ;
c) A. Gasco, V. Mortarini, G. Ruá, G. M. Nano, E. Menziani ibid. *9,* 577 (1972) ;
d) H. Wieland, E. Gmelin, Liebigs Ann. Chem. 367, 80 (1909) ;
e) O. Dimroth, O. Dienstbach, Ber. *41,* 4075 (1908) ;
f) G. Ponzio, Gazz. chim. ital. *66,* 819 (1936).

Die in der letztgenannten Publikation beschriebene Verbindung « Anilide del perossido dell' acido metilgliossimcarbonico » vom Schmelzpunkt 150 bis 151 °C besitzt nach dem heutigen Kenntnisstand eine der beiden Strukturen

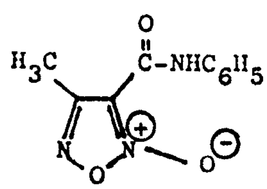

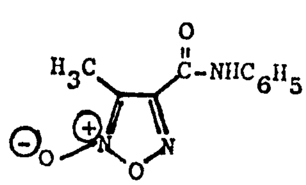

Diese Verbindung, die in der vorstehenden Tabelle unter der Nummer 26 aufgeführt ist, wird wie folgt

hergestellt : β-Methylbenzoylglyoxim der Formel

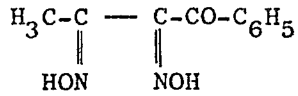

wird oxidiert, wobei eine Verbindung entsteht, die von G. Ponzio loc. cit. als « metilbenzoilperossido » bezeichnet wird, diese Verbindung wird mit Hydroxylamin-hydrochlorid in das «β-ossima del metilbenzoilperossido » überführt und dieses Oxim durch eine Beckmann-Umlagerung in das « Anilide des perossido dell' acido metilgliossimcarbonico » überführt.

Überraschenderweise wurde gefunden, daß die Verbindungen der Formel I bei relativ geringer Toxizität interessante und therapeutisch verwertbare pharmakodynamische Eigenschaften aufweisen. In Tierversuchen konnte gezeigt werden, daß die Verbindungen der Formel I bei kardiovaskulären Erkrankungen, wie Bluthochdruck und Angina pectoris, eingesetzt werden können. Die Verbindungen der Formel I bzw. sie enthaltende Arzneimittel können auch am Menschen zur Bekämpfung bzw. Verhütung von kardiovaskulären Krankheiten, wie Bluthochdruck und Angina pectoris, eingesetzt werden.

Unter den Verbindungen der Formel I werden das 1,2,5-Oxdiazol-2-oxid-3-methyl-4-carbonsäureamid ($R^1 = CH_3$, $R^2 = CONH_2$), vor allem das 1,2,5-Oxdiazol-2-oxid-4-methyl-3-carbonsäureamid ($R^1 = CONH_2$, $R^2 = CH_3$) und ganz besonders das 1,2,5-Oxdiazol-2-oxid-3,4-dicarbonsäure-diamid ($R^1 = R^2 = CONH_2$) bevorzugt.

Die antianginöse Wirkung der Verbindungen wurde nach der folgenden Methode gemessen :

Die Untersuchungen wurden an Bastardhunden beiderlei Geschlechts in Pentobarbital-Narkose (30 bis 40 mg/kg i. v.), oder in Urethan-Chloralose-Narkose (3 ml/kg Urethan-Chloralose-Gemisch i. v. = 20 mg/kg Chloralose und 250 mg/kg Urethan) durchgeführt. Die Beatmung der Tiere erfolgte mit einem Bird-Mark-7-Respirator. Der endexpiratorische Kohlensäuregehalt (gemessen mit dem Uras) betrug zwischen 4,5 und 5 Vol.%.

Während des gesamten Versuchs erhielten die Tiere mit Pentobarbital-Narkose eine Dauerinfusion von Pentobarbital i. v. : 4 mg/kg/6 ml/h, um eine konstante Narkosetiefe zu gewährleisten ; die Tiere mit Urethan-Chloralose-Narkose erhielten keine Dauerinfusion. Die Infusion wurde durch die Vena cephalica gegeben.

Nach der Präparation des Versuchstieres wurde ca. 1 Stunde gewartet bis sich alle haemodynamischen Parameter eingestellt hatten (steady state). Danach wurde mit dem eigentlichen Versuch begonnen.

Der mittlere Blutdruck wurde peripher in der Arteria femoralis über einen Statham-Druckaufnehmer gemessen.

Ein über die Arteria carotis in den linken Ventrikel geschobener Millar-Tip-Katheter lieferte das Signal für den linksventrikulären enddiastolischen Druck und die Herzfrequenz. Mit einem zweiten, über die Vena jugularis eingeschobenen Tip-Katheter wurde der mittlere Blutdruck in der Arteria pulmonalis erfaßt.

Bei dieser Untersuchungsmethode wurden beispielsweise für 1,2,5-Oxdiazol-2-oxid-3,4-dicarbonsäure-diamid (0,1 mg/kg i. d.) die folgenden Ergebnisse erhalten :

| Parameter | Vorwert (mbar) | Änderung (mbar) | Wirkungsdauer (Minuten) |
|---|---|---|---|
| Pulmonal-Arteriendruck | 21 | − 2,33 | 80 |
| linksventrikulärer endiastolischer Druck | 9 | − 3,33 | 80 |
| mittlerer Blutdruck | 136 | − 50 | 90 |
| Herzfrequenz (Schläge/Minute) | 101 | + 5 | |

Das 1,2,5-Oxdiazol-2-oxid-4-methyl-3-carbonsäureamid zeigte in der Dosierung von 0,1 mg/kg (i. v.) die folgenden Wirkungen :

| Parameter | Vorwert (mbar) | Änderung (mbar) | Wirkungsdauer (Minuten) |
|---|---|---|---|
| Pulmonal-Arteriendruck | 18 | − 2,26 | 20 |
| linksventrikulärer endiastolischer Druck | 6,7 | − 3,73 | 40 |
| mittlerer Blutdruck | 153 | − 33,3 | 60 |
| Herzfrequenz (Schläge/Minute) | 155 | 0 | |

Die akute Toxizität von 1,2,5-Oxdiazol-2-oxid-3,4-dicarbonsäurediamid wurde an der Maus zu $DL_{50}$ = 167 mg/kg i. v. ermittelt.

Auch bei der Ratte zeigt das 1,2,5-Oxdiazol-2-oxid-3,4-dicarbonsäureamid eine ausgeprägte kardiovaskuläre Wirkung. Die Blutdruckmessung erfolgte blutig über einen in die linke Arteria carotis chronisch implantierten Katheter an wachen spontan hypertonen Ratten.

| Dosis<br>10 mg/kg p. o. | systolischer Blutdruck (mbar) | diastolischer Blutdruck (mbar) |
|---|---|---|
| Vorwert | 247 | 206 |
| Änderung | – 56 | – 60 |
| Wirkdauer | 90 Minuten | 90 Minuten |

Die Verbindungen der Formel I können als Heilmittel, z. B. in Form pharmazeutischer Präparate, Verwendung finden. Die pharmazeutischen Präparate können oral, z. B. in Form von Tabletten, Lacktabletten, Dragees, Hart- und Weichgelatinekapseln, Lösungen, Sirupen, Emulsionen oder Suspensionen verabreicht werden. Die Verabreichung kann aber auch rektal, z. B. in Form von Suppositorien, oder parenteral, z. B. in Form von Injektionslösungen, oder perkutan, z. B. in Form von Salben, erfolgen.

Zur Herstellung von Tabletten, Lacktabletten, Dragees und Hartgelatinekapseln können die Verbindungen der Formel I mit pharmazeutisch inerten, anorganischen oder organischen Trägerstoffen verarbeitet werden. Als solche kann man für Tabletten, Dragees und Hartgelatinekapseln, z. B. Lactose, Maisstärke oder Derivate davon, Talk, Stearinsäure oder deren Salze etc. verwenden. Trägerstoffe für Weichgelatinekapseln und Suppositorien sind z. B. Fette, Wachse, halbfeste und flüssige Polyole, natürliche oder gehärtete Öle etc. Als Trägerstoffe für die Herstellung von Lösungen und Sirupen eignen sich z. B. Wasser, Saccharose, Invertzucker, Glukose, Polyole etc. Als Trägerstoffe für die Herstellung von Injektionslösungen eignen sich z. B. Wasser, Alkohole, Glyzerin, Polyole, pflanzliche Öle etc.

Die pharmazeutischen Präparate können daneben noch andere Stoffe, wie z. B. Sprengmittel, Konservierungsmittel, Lösevermittler, Stabilisierungsmittel, Netzmittel, Emulgiermittel, Süßmittel, Färbemittel, Aromatisierungsmittel, Salze zur Veränderung des osmotischen Drucks, Puffer, Überzugsmittel oder Antioxidantien enthalten. Sie können auch zwei oder mehrere Verbindungen der Formel I und/oder noch andere therapeutisch wertvolle Stoffe enthalten.

Erfindungsgemäß werden die Verbindungen der Formel I am Menschen bei der Bekämpfung bzw. Vorbeugung von Erkrankungen des kardiovaskulären Systems verwendet, beispielsweise als antihypertensive Wirkstoffe bei den verschiedenen Formen des Bluthochdrucks, bei der Bekämpfung bzw. Vorbeugung von Angina pectoris usw. Die Dosierung kann innerhalb weiter Grenzen variieren und ist in jedem einzelnen Fall den individuellen Gegebenheiten anzupassen. Im allgemeinen ist bei oraler Verabreichung eine tägliche Dosis von 0,1 bis 100 mg, vorzugsweise von 1 bis 20 mg, des Wirkstoffes pro menschlichem Individuum angemessen ; auch bei anderen Applikationsformen liegt die tägliche Dosis in ähnlichen Bereichen, d. h. ebenfalls im allgemeinen bei 0,1 bis 100 mg Wirkstoff pro menschlichem Individuum. Die tägliche Verabreichungsdosis wird normalerweise in mehrere, z. B. 2 bis 4 Teilverabreichungen aufgeteilt, wobei eine Einzeldosis 0,001 bis 1 mg Wirksubstanz/kg Körpergewicht enthält. Die Konzentration der Wirksubstanz in den pharmazeutischen Zubereitungen ist normalerweise 0,01 bis 20 %, vorzugsweise 0,05 bis 10 %, bezogen auf das Gesamtgewicht der Zubereitung.

In den nachfolgenden Beispielen, welche die vorliegende Erfindung illustrieren, ihren Umfang jedoch in keiner Weise beschränken sollen, werden pharmazeutische Präparate beschrieben, welche als Wirkstoff das 1,2,5-Oxdiazol-2-oxid-3,4-dicarbonsäurediamid enthalten.

Beispiel 1

Gelatineweichkapseln, enthaltend 5 mg Wirkstoff pro Kapsel :

| | pro Kapsel |
|---|---|
| Wirkstoff | 5 mg |
| Aus Kokosfett fraktioniertes Triglyceridgemisch | 150 mg |
| Kapselinhalt | 155 mg |

Beispiel 2

Injektionslösung, enthaltend 1 mg Wirkstoff pro ml :

| | pro ml |
|---|---|
| Wirkstoff | 1,0 mg |
| Polyäthylenglycol 400 | 0,3 ml |

| | |
|---|---|
| Natriumchlorid | 2,7 mg |
| Wasser zu Injektionszwecken ad | 1 ml |

Beispiel 3

Emulsionen, enthaltend 3 mg Wirkstoff pro 5 ml :

| | pro 100 ml Emulsion |
|---|---|
| Wirkstoff | 0,06 g |
| Neutralöl | q.s. |
| Natriumcarboximethylcellulose | 0,6 g |
| Polyoxiäthylen-stearat | q.s. |
| Glycerin rein | 0,2 bis 2,0 g |
| Geschmacksstoff | q.s. |
| Wasser (entsalzt oder destilliert) ad | 100 ml |

Beispiel 4

Rektale Arzneiform, enthaltend 4 mg Wirkstoff pro Suppositorium

| | pro Suppositorium |
|---|---|
| Wirkstoff | 4 mg |
| Suppositoriengrundmasse ad | 2 g |

Beispiel 5

Tabletten, enthaltend 2 mg Wirkstoff pro Tablette

| | pro Tablette |
|---|---|
| Wirkstoff (feingemahlen) | 2 mg |
| Maisstärke (weiß) | 150 mg |
| Milchzucker | 60 mg |
| Mikrokristalline Cellulose | 50 mg |
| Polyvinylpyrrolidon | 20 mg |
| Magnesiumstearat | 2 mg |
| Natriumcarboximethylstärke | 25 mg |
| | 309 mg |

**Ansprüche**

1. 1,2,5-Oxdiazol-2-oxide der allgemeinen Formel I

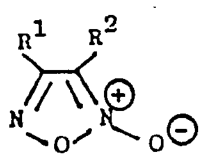

(I)

worin $R^1$ und $R^2$ die folgende Bedeutung besitzen :

| | $R^1$ | $R^2$ |
|---|---|---|
| 1. | $CO—NH_2$ | $CH_3$ |
| 2. | $COOC_2H_5$ | $CH_3$ |
| 3. | $CO—NHNH_2$ | $CH_3$ |
| 4. | $COOH$ | $CH_3$ |
| 5. | $—NH—COOC_2H_5$ | $CH_3$ |
| 6. | $—NH—COOC_3H_7(n)$ | $CH_3$ |
| 7. | $—NH—COOC_3H_7(i)$ | $CH_3$ |
| 8. | $—NH—COOC_4H_9(n)$ | $CH_3$ |

| | |
|---|---|
| 9. $—NH—COOCH_2$-phenyl | $CH_3$ |
| 10. $CO—NH_2$ | $CO—NH_2$ |
| 11. CN | CN |
| 12. COOH | $COOCH_3$ |
| 13. $COOCH_3$ | $COOCH_3$ |
| 14. $COOC_2H_5$ | $COOC_2H_5$ |
| 15. $CH_3$ | $CO—NH_2$ |
| 16. $CH_3$ | $COOC_2H_5$ |
| 17. $CH_3$ | $CO—NHNH_2$ |
| 18. $CH_3$ | COOH |
| 19. CN | $CO—NH_2$ |
| 20. CO—NH-phenyl | CO—NH-phenyl |
| 21. $CH_3$ | $NH—COOC_2H_5$ |
| 22. $CH_3$ | $NH—COOC_3H_7(n)$ |
| 23. $CH_3$ | $NH—COOC_3H_7(i)$ |
| 24. $CH_3$ | $NH—COOC_4H_9(n)$ |
| 25. $CH_3$ | $NH—COOCH_2$-phenyl |
| 26. CO—NH-phenyl | $CH_3$ |
| 27. $CH_3$ | CO—NH-phenyl |

zur Verwendung bei der Bekämpfung oder der Vorbeugung von kardiovaskulären Erkrankungen.

2. 1,2,5-Oxdiazol-2-oxid-3,4-dicarbonsäure-diamid zur Verwendung bei der Bekämpfung oder der Vorbeugung von kardiovaskulären Erkrankungen.

3. 1,2,5-Oxdiazol-2-oxid-3,4-dicarbonsäure-diamid zur Verwendung bei der Bekämpfung oder der Vorbeugung von Angina-pectoris.

4. 1,2,5-Oxdiazol-2-oxid-3,4-dicarbonsäure-diamid zur Verwendung bei der Bekämpfung oder der Vorbeugung des Bluthochdrucks.

5. 1,2,5-Oxdiazol-2-oxid-3-methyl-4-carbonsäureamid zur Verwendung bei der Bekämpfung oder der Vorbeugung von kardiovaskulären Erkrankungen, insbesondere von Angina-pectoris.

6. 1,2,5-Oxdiazol-2-oxid-4-methyl-3-carbonsäureamid zur Verwendung bei der Bekämpfung oder der Vorbeugung von kardiovaskulären Erkrankungen, insbesondere von Angina-pectoris.

7. 1,2,5-Oxdiazol-2-oxid-3-methyl-4-carbonsäureamid zur Verwendung bei der Bekämpfung oder der Vorbeugung des Bluthochdrucks.

8. 1,2,5-Oxdiazol-2-oxid-4-methyl-3-carbonsäureamid zur Verwendung bei der Bekämpfung oder der Vorbeugung des Bluthochdrucks.

9. Arzneimittel, insbesondere kardiovaskulär wirksames Arzneimittel, dadurch gekennzeichnet, daß es mindestens eine Verbindung der Formel I des Anspruchs 1, vorzugsweise das 1,2,5-Oxdiazol-2-oxid-3,4-dicarbonsäure-diamid, enthält.

10. Verfahren zur Herstellung von pharmazeutischen Präparaten, die zur Bekämpfung oder Vorbeugung kardiovaskulärer Erkrankungen bestimmt sind, dadurch gekennzeichnet, daß eine oder mehrere Verbindungen der Formel I des Anspruchs 1 mit pharmazeutisch inerten Trägerstoffen kombiniert werden.

## Claims

1. 1,2,5-Oxadiazole-2-oxides of the general formula I

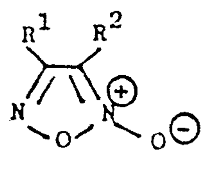

(I)

wherein $R^1$ and $R^2$ have the following meaning :

| $R^1$ | $R^2$ |
|---|---|
| 1. $CO—NH_2$ | $CH_3$ |
| 2. $COOC_2H_5$ | $CH_3$ |
| 3. $CO—NHNH_2$ | $CH_3$ |
| 4. COOH | $CH_3$ |
| 5. $—NH—COOC_2H_5$ | $CH_3$ |
| 6. $—NH—COOC_3H_7(n)$ | $CH_3$ |
| 7. $—NH—COOC_3H_7(i)$ | $CH_3$ |

| | | |
|---|---|---|
| 8. | $-NH-COOC_4H_9(n)$ | $CH_3$ |
| 9. | $-NH-COOCH_2$-phenyl | $CH_3$ |
| 10. | $CO-NH_2$ | $CO-NH_2$ |
| 11. | CN | CN |
| 12. | COOH | $COOCH_3$ |
| 13. | $COOCH_3$ | $COOCH_3$ |
| 14. | $COOC_2H_5$ | $COOC_2H_5$ |
| 15. | $CH_3$ | $CO-NH_2$ |
| 16. | $CH_3$ | $COOC_2H_5$ |
| 17. | $CH_3$ | $CO-NHNH_2$ |
| 18. | $CH_3$ | COOH |
| 19. | CN | $CO-NH_2$ |
| 20. | CO—NH-phenyl | CO—NH-phenyl |
| 21. | $CH_3$ | $NH-COOC_2H_5$ |
| 22. | $CH_3$ | $NH-COOC_3H_7(n)$ |
| 23. | $CH_3$ | $NH-COOC_3H_7(i)$ |
| 24. | $CH_3$ | $NH-COOC_4H_9(n)$ |
| 25. | $CH_3$ | $NH-COOCH_2$-phenyl |
| 26. | CO—NH-phenyl | $CH_3$ |
| 27. | $CH_3$ | CO—NH-phenyl |

for use in combating or preventing cardiovascular diseases.

2. 1,2,5-Oxadiazole-2-oxide-3,4-dicarboxylic acid diamide for use in combating or preventing cardiovascular diseases.

3. 1,2,5-Oxadiazole-2-oxide-3,4-dicarboxylic acid diamide for use in combating or preventing angina pectoris.

4. 1,2,5-Oxadiazole-2-oxide-3,4-dicarboxylic acid diamide for use in combating or preventing high blood pressure.

5. 1,2,5-Oxadiazole-2-oxide-3-methyl-4-carboxylic acid amide for use in combating or preventing cardiovascular diseases, in particular angina pectoris.

6. 1,2,5-Oxadiazole-2-oxide-4-methyl-3-carboxylic acid amide, for use in combating or preventing cardiovascular diseases, in particular angina pectoris.

7. 1,2,5-Oxadiazole-2-oxide-3-methyl-4-carboxylic acid amide for use in combating or preventing high blood pressure.

8. 1,2,5-Oxadiazole-2-oxide-4-methyl-3-carboxylic acid amide for use in combating or preventing high blood pressure.

9. Medicament, in particular medicament having a cardiovascular activity, characterised in that it contains at least one compound of the formula I, of Claim 1, preferably 1,2,5-oxadiazole-2-oxide-3,4-dicarboxylic acid diamide.

10. Process for preparing pharmaceutical formulations for use in combating or preventing cardiovascular diseases, characterised in that one or several compounds of formula I, of Claim 1, are combined with pharmaceutically inert excipients.

**Revendications**

1. L'utilisation, pour le traitement ou la prévention de maladies cardio-vasculaires, des 1,2,5-oxadiazole-2-oxydes de formule générale I

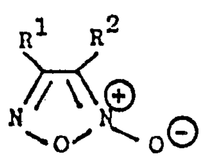

(I)

dans laquelle les symboles $R^1$ et $R^2$ ont l'une des significations suivantes :

| | $R^1$ | $R^2$ |
|---|---|---|
| 1. | $CO-NH_2$ | $CH_3$ |
| 2. | $COOC_2H_5$ | $CH_3$ |
| 3. | $CO-NHNH_2$ | $CH_3$ |
| 4. | COOH | $CH_3$ |
| 5. | $-NH-COOC_2H_5$ | $CH_3$ |
| 6. | $-NH-COOC_3H_7(n)$ | $CH_3$ |

| | | |
|---|---|---|
| 7. | $-NH-COOC_3H_7(i)$ | $CH_3$ |
| 8. | $-NH-COOC_4H_9(n)$ | $CH_3$ |
| 9. | $-NH-COOCH_2$-phényle | $CH_3$ |
| 10. | $CO-NH_2$ | $CO-NH_2$ |
| 11. | CN | CN |
| 12. | COOH | $COOCH_3$ |
| 13. | $COOCH_3$ | $COOCH_3$ |
| 14. | $COOC_2H_5$ | $COOC_2H_5$ |
| 15. | $CH_3$ | $CO-NH_2$ |
| 16. | $CH_3$ | $COOC_2H_5$ |
| 17. | $CH_3$ | $CO-NHNH_2$ |
| 18. | $CH_3$ | COOH |
| 19. | CN | $CO-NH_2$ |
| 20. | CO—NH-phényle | CO—NH-phényle |
| 21. | $CH_3$ | $NH-COOC_2H_5$ |
| 22. | $CH_3$ | $NH-COOC_3H_7(n)$ |
| 23. | $CH_3$ | $NH-COOC_3H_7(i)$ |
| 24. | $CH_3$ | $NH-COOC_4H_9(n)$ |
| 25. | $CH_3$ | $NH-COOCH_2$-phényle |
| 26. | CO—NH-phényle | $CH_3$ |
| 27. | $CH_3$ | CO—NH-phényle |

2. Utilisation du 1,2,5-oxadiazole-2-oxyde-3,4-dicarboxamide dans le traitement ou la prévention de maladies cardio-vasculaires.

3. Utilisation du 1,2,5-oxadiazole-2-oxyde-3,4-dicarboxamide dans le traitement ou la prévention de l'angine de poitrine.

4. Utilisation du 1,2,5-oxadiazole-2-oxyde-3,4-dicarboxamide dans le traitement ou la prévention de l'hypertension.

5. Utilisation du 1,2,5-oxadiazole-2-oxyde-3-méthyl-4-carboxamide dans le traitement ou la prévention de maladies cardio-vasculaires, en particulier de l'angine de poitrine.

6. Utilisation du 1,2,5-oxadiazole-2-oxyde-4-méthyl-3-carboxamide dans le traitement ou la prévention de maladies cardio-vasculaires, en particulier de l'angine de poitrine.

7. Utilisation du 1,2,5-oxadiazole-2-oxyde-3-méthyl-4-carboxamide dans le traitement ou la prévention de l'hypertension.

8. Utilisation du 1,2,5-oxadiazole-2-oxyde-4-méthyl-3-carboxamide dans le traitement ou la prévention de l'hypertension.

9. Médicament, en particulier médicament doué d'actions cardio-vasculaires, caractérisé en ce qu'il contient au moins un composé de formule I selon la revendication 1, de préférence le 1,2,5-oxadiazole-2-oxyde-3,4-dicarboxamide.

10. Procédé de fabrication de préparations pharmaceutiques pour le traitement ou la prévention de maladies cardio-vasculaires, procédé caractérisé en ce que l'on associe un ou plusieurs composés de formule I selon la revendication 1 avec des véhicules pharmaceutiquement inertes.